# Europäisches Patentamt
# European Patent Office
# Office européen des brevets

(11) Veröffentlichungsnummer: **0 108 231**
**B1**

(12) **EUROPÄISCHE PATENTSCHRIFT**

(45) Veröffentlichungstag der Patentschrift:
**25.02.87**

(21) Anmeldenummer: **83109442.0**

(22) Anmeldetag: **22.09.83**

(51) Int. Cl.⁴: **C 12 N 11/08, C 12 P 33/00 //**
**C12R1/06, C12R1/66, C12R1/07,**
**C12R1/20, C12R1/32, C12R1/85**

(54) Biokatalysator.

(30) Priorität: **09.11.82 DE 3241829**

(43) Veröffentlichungstag der Anmeldung:
**16.05.84 Patentblatt 84/20**

(45) Bekanntmachung des Hinweises auf die Patenterteilung:
**25.02.87 Patentblatt 87/9**

(84) Benannte Vertragsstaaten:
**AT BE CH DE FR GB IT LI LU NL SE**

(56) Entgegenhaltungen:
**DD-A-152 580**
**DD-A-152 581**
**DE-A-2 353 102**
**DE-A-2 729 490**
**GB-A-1 555 004**
**GB-A-2 080 669**
**US-A-3 821 083**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT**
**Berlin und Bergkamen, Müllerstrasse 170/178**
**Postfach 65 03 11, D-1000 Berlin 65 (DE)**

(72) Erfinder: **Manecke, Georg, Prof. Dr.,**
**Hittorfstrasse 29, D-1000 Berlin 33 (DE)**
Erfinder: **Klussmann, Udo, Dipl.- Chem.,**
**Zimmermannstrasse 18, D-1000 Berlin 41 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

2

## Beschreibung

Die Erfindung betrifft den in den Patentansprüchen gekennzeichneten Biokatalysator, ein Verfahren zu seiner Herstellung und seine Verwendung zur mikrobiologischen Umwandlung von Steroiden.

Bekanntlich werden Mikroorganismen-Kulturen häufig zur Steroid-Umwandlung verwendet. So dienen Kulturen der Species Arthrobacter simplex ATCC 6946, 13260 und IFO 3530 oder Bacillus sphaericus ATCC 7054, 7055, 12488 und 13805 zur $\Delta^1$-Dehydrierung von 3-Keto-$\Delta^4$-steroiden. Kulturen von Flavobacterium dehydrogenans ATCC 13930 werden unter anderem zur Umwandlung von 3β-Hydroxy-$\Delta^5$-steroiden in 3- Keto-$\Delta^4$-steroide verwendet. Zur Reduktion von Ketosteroiden eignen sich beispielsweise Kulturen von Saccharomyces üvarum (NCYC 91 oder CBS 1508). Des weiteren sei der Seitenkettenabbau von Steroiden erwähnt, der unter anderem mit Kulturen von Mycobacterium spec. (NRRL-B 3683 und NRRL-B 3805) durchgeführt werden kann. Letzlich wäre noch die 11α- und 11β-Hydroxylierung von Steroiden zu erwähnen, welche mit Hilfe von Kulturen der Species Aspergillus occhraceus (CBS 13252, ATCC 1008, ATCC 12 337, ATCC 18500 oder NRRL 405) beziehungsweise mit Curvularia lunata (NRRL 2380, NRRL 2434, ATCC 12017 oder IFO 6286) bewirkt werden kann.

Mithilfe des erfindungsgemäßen Verfahrens kann man aus den obengenannten Mikroorganismen Biokatalysatoren herstellen, in denen die Mikroorganismen in immobilisierter aber vermehrungsfähiger Form vorliegen. Gegenüber anderen Biokatalysatoren, bei denen Mikroorganismen in Polymeren fixiert sind, zeichnen sich die erfindungsgemäßen Biokatalysatoren durch eine überlegene Wirksamkeit aus.

Die für die Herstellung des Biokatalysators benötigten Mikroorganismen werden in üblicher Weise angezüchtet und nach erfolgter Anzucht abfiltriert oder abzentrifugiert, mit Wasser oder verdünnter Pufferlösung gewaschen und nochmals filtriert oder zentrifugiert. Die so erhaltene feuchte Biomasse kann ohne weitere Aufarbeitung zur Herstellung des Biokatalysators verwendet werden, wobei man etwa die 0,5 bis 7-fache Menge feuchte Biomasse bezogen auf das Acrolein-(1-Vinyl-2-pyrrolidon)-Copolymerisat (Trockenmasse) verwendet. Andererseits kann aber diese Biomasse auch durch Sprühtrocknung getrocknet werden und man setzt dann 0,1 bis 2,0 mal so viel Trockenpulver wie Copolymerisat (Trockenmasse) ein.

Das für den Biokatalysator verwendete Acrolein-(1-Vinyl-2-pyrrolidon)-Copolymerisat kann nach dem im Patentanspruch 6 gekennzeichneten Verfahren hergestellt werden. Bei diesem Verfahren verwendet man als Initiator vorzugsweise Peroxide, wie zum Beispiel Wasserstoffperoxid, Benzoylperoxid, Cumolhydroperoxid, Di-tert.-butylperoxid oder insbesondere Kaliumperoxodisulfat. Das Verfahren wird bei einer Temperatur von 10 bis 40° C und insbesondere bei einer Temperatur von 15 bis 30° C durchgeführt. Am problemlosesten läßt sich die Reaktion bei Raumtemperatur durchführen.

Weitgehend unabhängig davon wieviel Mol Acrolein man pro Mol 1-Vinyl-2-pyrrolidon verwendet ist das Copolymerisat aus etwa äquimolaren Mengen beider Komponenten zusammengesetzt, wie man mittels Aldehydgruppenbestimmung und Elementaranalyse nachweisen kann. Zur Erzielung guter Ausbeuten an Copolymerisat ist es aber zweckmäßig etwa 0,5 bis 1,5 Mol Acrolein pro Mol 1-Vinyl-2-pyrrolidon zur Reaktion einzusetzen. Zweckmäßigerweise führt man die Polymerisation in einer wässrigen Lösung durch die 2 bis 20 % des Acrolein-(1-Vinyl-2-pyrrolidon)-Gemisches enthält. Pro Mol Acrolein-(1-Vinyl-2-pyrrolidon-Gemisch verwendet man vorzugsweise 0,5 bis 10 und insbesondere 1 bis 4 Mol Prozent des Initiators. Nach erfolgter Polymerisation werden die Monomeren und der Initiator vorzugsweise durch Dialyse entfernt und die erhaltene Lösung des Copolymerisats kann direkt zur Herstellung des Biokatalysators verwendet werden. Andererseits ist es selbstverständlich auch möglich, die Copolymerisat-Lösung vor dem Gebrauch zu verdünnen oder aber beispielsweise mittels Gefriertrocknung zu konzentrieren.

Die Alkylendioxydiamine sind bekannt oder können in an sich bekannter Weise hergestellt werden. (J. Chem. Soc. 1947, 963 ff). Unter den Alkylendioxydiaminen sind solche besonders bevorzugt, die 5 bis 9 Kohlenstoffatome besitzen. Da die längerkettigen Alkylendioxydiamine in Wasser schwer löslich sind, ist es zweckmäßig zur Herstellung der Biokatalysatoren deren Salze (zum Beispiel die Monochloride oder Monosulfate) zu verwenden.

Zur Herstellung des Biokatalysators werden die Mikroorganismen in der Acrolein-(1-Vinyl-2-pyrrolidon)-Copolymerisat-Lösung suspendiert, wobei man noch zusätzlich bis zu 25% eines inerten Lösungsmittel - wie zum Beispiel Dimethylformamid oder Dimethylsulfoxid - zusetzen kann. Wenn eine gleichmäßige Suspension entstanden ist, setzt man soviel Alkylendioxydiamin unter Rühren zu, daß das Verhältnis von Oxyaminogruppen im Vernetzer und Aldehydgruppen im Copolymeren vorzugsweise 0,5 bis 1,5 insbesondere aber 1:1 beträgt. Danach wird die Mischung mit einer Pufferlösung oder mit Lauge auf einen pH-Wert von 3 bis 7 eingestellt, der Biokatalysator abgetrennt und zerkleinert. Es ist zweckmäßig den Biokatalysator naß zu sieben und die Siebfraktionen von 0,02 bis 2 mm vorzugsweise solche von 0,05 bis 1,5 mm Korngröße zur Umsetzung von Steroiden zu verwenden.

Wässrige Suspensionen des Biokatalysators können in der gleichen Weise zur Steroidtransformation verwendet werden, wie

2

die entsprechenden Suspensionen der Mikroorganismen selbst. So werden zunächst mittels der üblichen Vorversuche die günstigsten Fermentationsbedingungen, wie der Substratkonzentration, der technischen Bedingungen wie Temperatur, Belüftung, pH-Wert und der optimalen Fermentationszeit ermittelt.

In einer besonders bevorzugten Ausführungsform setzt man der Suspension des Biokatalysators mikronisiertes Steroid zu, fermentiert und trennt nach erfolgter Umsetzung das feindisperse Produkt durch Siebung von grobkörnigen Biokatalysator. Letzterer kann dann - gegebenenfalls nachdem man ihn durch Behandeln mit einer Nährlösung reaktiviert hat - erneut zur Steroidtransformation verwendet werden.

Die nachfolgenden Ausführungsbeispiele dienen zur Erläuterung des erfindungsgemäßen Verfahrens zur Herstellung des Biokatalysators und zur näheren Erläuterung von dessen Anwendung zur Steroidumwandlung.

**Beispiel 1**

a) Zwei 2 l Erlenmeyerkolben, von denen jeder 500 ml einer sterilisierten Nährlösung aus 0,5 % Corn steep, 0,1 Hefeextrakt und 0,05 % Stärkezucker, eingestellt auf pH 7,0 enthält, wird mit einer Lyophilkultur von Arthrobacter simplex (ATCC 6946) beimpft und 48 Stunden bei 30° C auf einem Rotationsschüttler geschüttelt. Diese Vorkultur dient zur Beimpfung eines 50 l Glasfermenters, der mit 30 l eines sterilisierten Mediums aus 0,5 % Corn steep, 0,1 % Hefeextrakt und 0,05 % Stärkezucker, eingestellt auf pH 7,0, gefüllt ist. Nach der Beimpfung wird bei 29° C unter Belüftung (10 Liter/Min.), 0,7 atü Druck und Rühren (220 Umdrehungen pro Minute) 24 Stunden germiniert. Mit dieser Vorfermenter-Kultur wird nun ein Stahlfermenter beimpft, der 500 l eines bei 121° C und 1,1 atü sterilisierten Mediums aus 0,5 % Corn steep, 0,1 % Hefeextrakt und 0,05 % Stärkezucker, eingestellt auf pH 7,0, enthält und unter den Bedingungen des Vorfermenters ebenfalls 24 Stunden bis zur optimalen Zelldichte germiniert.

Anschließend wird die Kultur in einem Labor Separator LG 205 [Firma Westfalia Separator AG/Oelde (Westf.)] bei 10 000 Umdrehungen/Min. separiert. Die abgeschleuderte Zellmasse wird zur Entfernung anhaftender Bestandteile des Nährmediums mit destillierten Wasser gewaschen und erneut zentrifugiert.

Die auf diese Weise dargestellte feuchte Bakterienpaste (2,85 kg) wird in 2,85 l eines 0,05 molaren Tris-(hydroxymethyl)-methylamin-Puffers von pH 7,6 aufgeschlemmt, zur Zerteilung von Zellklumpen durch in Metallgazefilter gedrückt und in einem Universal-Labor-Zerstäubungstrockner [Firma Zahn & Co.,

Hameln, Deutschland] bei einer Verdampfungstemperatur von 80° C versprüht, wobei 600 ml Zellsuspension pro Stunde eingespeist werden.

Man erhält so 513 g Arthrobacter simplex Trockenpulver.

b) 44,9 g redestilliertes Acrolein und 133,4 g redestilliertes 1-Vinyl-2-pyrrolidon werden unter Stickstoff in einem Liter Wasser gelöst, mit 10,8 g Kaliumperoxodisulfat versetzt, gerührt bis das Salz gelöst ist und dann 24 Stunden bei Raumtemperatur stehengelassen.

Dann dialysiert man das Reaktionsgemisch 3 Tage lang gegen destilliertes Wasser und erhält die Acrolein-(1-Vinyl-2-pyrrolidon)-Copolymerisat-Lösung mit einem Gehalt von 43,5 mg Copolymerisat pro ml Lösung. Der Aldehydgruppengehalt des Copolymerisats beträgt 5,32 mMol pro Gramm.

c) 228 ml der Acrolein-(1-Vinyl-2-pyrrolidon)-Copolymerisat-Lösung (enthaltend 10 g Copolymerisat), werden mit 22 ml Dimethylformamid versetzt. Dann gibt man zu der Lösung unter kräftigem Rühren 10 g Arthrobacter simplex Trockenpulver und nachdem dieses gleichmäßig verteilt ist, 5,32 ml einer 0,5 molaren wässrigen Lösung von 1,6-Bis-aminooxy-hexan, Monohydrochlorid. Sobald der pH-Wert sinkt, wird er durch Zugabe von 1 n Natronlauge auf pH 4,5 eingestellt. 10 Minuten später füllt man das Reaktionsgemisch mit 0,05 mol Phosphatpuffer (pH 6,86) auf 2,5 l auf, rührt noch eine Stunde, zerkleinert das erhaltene Produkt, wäscht es mit dem Phosphat-Puffer und siebt es naß. Die Siebfraktionen mit einer Korngröße von 0,1 bis 0,5 mm werden in feuchten Zustand aufbewahrt und zur Steroidumwandlung verwendet. Der feuchte Biokatalysator enthält 14 % Trockenmasse.

d) 440 mg des feuchten Biokatalysators werden in 50 ml 0,05 molarem Phosphatpuffer von pH 6,86, suspendiert, mit 180 mg mikronisiertem Hydrocortison versetzt und 24 Stunden lang bei Raumtemperatur geschüttelt. Dann filtriert man den Biokatalysator ab und bestimmt mittels Differential- Puls-Polagrophaphie den Gehalt an gebildetem Prednisolon. Die Ausbeute beträgt 87 % der Theorie.

Der abfiltrierte Biokatalysator kann abermals zur Umsetzung von Hydrocortison verwendet werden.

**Beispiel 2**

a) 106,4 g 1,8-Dibromoktan werden in 400 ml absolutem Ethanol gelöst, mit 82,2 g Hydroxycarbamidsäureethylester und 43,8 g Kaliumhydroxid versetzt und 6 Stunden lang unter Rückfluß erhitzt. Man engt die Reaktionsmischung im Vakuum ein, verdünnt den Rückstand mit Wasser, extrahiert mit Diethylether und engt den Etherextrakt ein.

Der erhaltene Rückstand wird mit einer Lösung von 40 g Kaliumhydroxid in 80 ml Wasser versetzt und 2,5 Stunden lang unter Rückfluß erhitzt.

Man läßt die Reaktionsmischung erkalten, extrahiert sie mit Diethylether und fraktioniert die organische Phase. Man erhält so 16,43 g 1,8-Di-aminooxy-oktan vom Siedepunkt 115° C/0,4 torr.

16,43 g Di-aminooxy-oktan werden unter Rühren mit 167,3 ml I n wässriger Salzsäure versetzt bis der pH-Wert der Lösung 2,4 beträgt. Dann engt man die Lösung im Vakuum ein und kristallisiert den Rückstand aus 93 %igem Ethanol um. Man erhält so 14,83 g Di-aminooxy-oktan-Monohydrochlorid vom Schmelzpunkt 174° C.

b) Unter den Bedingungen des Beispiels 1 c - jedoch unter Verwendung von der entsprechenden Menge 1,8-Di-amino-oxy-oktan, Monohydrochlorid wird ein Arthrobacter simplex (ATCC 6946) Biokatalysator hergestellt der ebenfalls die Fähigkeit hat, Hydrocortison zu Prednisolon zu dehydrieren.

## Beispiel 3

a) Unter den Bedingungen des Beispiels 1 a, jedoch unter Verwendung eines Nährmediums, das 0,5 % Corn steep liquor, 0,3 % Hefeextrakt und 0,2 % Stärkezucker enthält, werden 500 I einer Flavobacterium-dehydrogenans (ATCC 13930)-Kultur hergestellt und zu 731 g Flavobacterium-dehydrogenans Trockenpulver aufbereitet.

b) Unter den Bedingungen des Beispiels 1 c, jedoch unter Verwendung des Flavobacterium dehydrogenans Trockenpulver, wird ein Biokatalysator hergestellt, der die Fähigkeit hat 3β,17α,21-Trihydroxy-5-pregnen-20-on in 17α,21-Di-hydroxy-4-pregnen-3,20-dion umzuwandeln.

## Beispiel 4

a) Ein 2 I Erlenmeyerkolben, der 500 ml eines sterilisierten Nährmediums, bestehend aus 2 % Edamin, 5 % Dextrose und 0,3 % Corn steep liqour von pH 5,3 enthält, wird mit einer Lyophilkultur von Saccharomyces uvarum (NCYC 91) beimpft und 24 Stunden auf dem Rotationsschüttler bei 26° C geschüttelt.

Mit dieser Vorkultur wird nun ein 20 Liter Glasfermenter mit 15 Liter sterilem Kulturmedium (2 % Edamin, 5 % Dextrose und 0,3 % Corn steep liquor pH 5,3) beimpft und bei 26° C unter Belüftung mit steriler Luft 0,7 atü Druck und Rühren 60 Stunden germiniert.

Danach wird die Kulturbrühe in einem Labor-Separator zentrifugiert und das Zellsediment zur Entfernung von Medienbestandteilen mit 3 Liter destilliertem Wasser gewaschen und erneut abgeschleudert. Die auf diese Weise dargestellte Zellmasse wird in 0,05 molaren Phosphatpuffer nach Sörensen von pH 6,4, aufgeschlemmt und in einem Labor-Zerstäubungstrockner bei 80° C versprüht. In der Vorlage sammeln sich nach Beendigung der Sprühtrocknung 195 g Hefe-Trockenpulver.

b) Unter den Bedingungen des Beispiels 1 c, jedoch unter Verwendung des Hefe-Trockenpulvers wird ein Biokatalysator hergestellt, der die Fähigkeit besitzt, 4-Androsten-3,17-dion zum 17ß-Hydroxy-4-androsten-3-on zu reduzieren.

## Beispiel 5

a) Ein 750 ml Erlenmeyerkolben wird mit 200 ml einer sterilen Nährlösung, enthaltend 1 % Hefeextrakt, 0,45 % Dinatriumhydrogenphosphat, 0,34 % Kaliumdihydrogenphosphat und 0,2 % Tagat(R)02 - eingestellt auf pH 6,7 - beschickt, mit einer Abschwemmung einer Trockenkultur von Mycobacterium spec. NRRL-B-3805 beimpft und 3 Tage lang mit 190 Umdrehungen pro Minute bei 30° C geschüttelt.

Ein 50 I Fermenter mit 40 I einer sterilen Nährlösung, enthaltend 1,23 % Hefeextrakt (65 %ig), 068 % Kaliumdihydrogenphosphat und 0,2 % Tagat(R)02 - eingestellt auf pH 0,6 - wird mit 200 ml der Mycobacterium spec. Anzuchtskultur beimpft und die Vorkultur bei 30° C unter Belüftung (2 m³ pro Stunde) 48 Stunden lang inkubiert. Man zentrifugiert die Bakterienmasse ab, schleimt sie mit Wasser auf und zentrifugiert nochmals.

b) Unter den Bedingungen des Beispiels 1 c, jedoch unter Verwendung von 25 g Mycobacterium spec. Bakterienmasse wird ein Biokatalysator hergestellt, der die Fähigkeit hat 4-Cholesten-3-on in 4-Androsten-3,27-dion umzuwandeln.

## Beispiel 6

a) Ein 7 bis 14 Tage altes Bierwürze-Schrägröhrchen mit Curvularia lunata (NRRL 2380) wird mit 3 ml physiologischer Natriumchloridlösung abgeschwemmt und damit ein 2 I Erlenmeyerkolben beimpft, der 500 ml einer 30 Minuten bei 120° C im Autoklaven sterilisierten Nährlösung aus 2 % Glukose und 2 % Corn steep, eingestellt auf pH 6,5, enthält. Nach 60 Stunden Schütteln auf einem Rotationsschüttler bei 30° C dienen 250 ml dieser Anzuchtskultur zur Beimpfung des Vorfermenters. Ein 20 I Vorfermenter, der mit 15 I eines bei 121° C und I bar Überdruck sterilisierten Nährmediums der gleichen Zusammensetzung wie das Anzuchtmedium beschickt ist, wird mit 250 ml Anzuchtkultur beimpft. Unter Zugabe von Silicon SH als Antischaummittel wird nun bis 29° C und 0,6 bar Druck unter Belüftung (15 Liter/Min.) und Rühren (220 Umdrehungen/ Min.) 24 Stunden germiniert.

Dann saugt man das Pilzmycel ab, schleimt es in Wasser auf und saugt es nochmals ab.

b) Unter den Bedingungen des Beispiels 1 c jedoch unter Verwendung von 25 g Curvularia lunata Pilzmycel wird ein Biokatalysator hergestellt, der sich zur Umwandlung von 17α,21-Dihydroxy-4-pregnen-3,20-dion in Hydrocortison eignet.

**Beispiel 7**

a) Ein 2 l Erlenmeyerkolben mit 500 ml einer sterilen Nährlösung, enthaltend 1 % Corn steep liquor, 1,25 % Sojabohnenmehl und 0,005 % Sojaöl wird mit einer 10 Tage alten Schrägagarkultur von Aspergillus occhraceus ATCC 1008 beimpft und 72 Stunden lang bei 30° C und 165 Umdrehungen pro Minute geschüttelt.

Mit dieser Anzuchtskultur wird ein 20 l Glasfermenter - enthaltend 14,5 sterilisierter Nährlösung gleicher Zusammensetzung - beimpft und unter Rühren (220 Umdrehungen pro Minute) und Belüften (15 Liter pro Minute) bei 30° C germiniert.

Nach 24 Stunden Anwachsphase saugt man das Mycel ab, schleimt es in Wasser auf und saugt es nochmals ab.

b) Unter den Bedingungen des Beispiels 1 c, jedoch unter Verwendung von 25 g Aspergillus occhraceus Pilzmycel wird ein Biokatalysator hergestellt, der sich zur Umwandlung von 17α,21-Dihydroxy-4-pregnen-3,20-dion in 11α,17α,21-Trihydroxy-4-pregnen-3,20-dion eignet.

**Patentansprüche**

1. Biokatalysator enthaltend immobilisierte Mikroorganismen der Species Arthrobacter simplex, Aspergillus occhraceus, Bacillus sphaericus, Curvularia lunata, Flavobacterium dehydrogenans, Mycobacterium spec. oder Saccharomyces uvarum dadurch gekennzeichnet, daß die Mikroorganismen immobilisiert sind in einem Copolymerisat aus Acrolein und 1-Vinyl-2-pyrrolidon, welches durch Umsetzung mit einem Alkylendioxydiamin der allgemeinen Formel
$$H_2NO\text{-}(CH_2)_nONH_2$$
worin n die Ziffern 2 bis 12 bedeutet, vernetzt ist.

2. Biokatalysator gemäß Patentanspruch 1, dadurch gekennzeichnet, daß er 10 bis 75 Gewichtsprozent Mikroorganismen enthält.

3. Biokatalysator gemäß Patentanspruch 1 und 2, dadurch gekennzeichnet daß er eine Korngröße von 0,05 bis 1 mm hat.

4. Verfahren zur Herstellung von Biokatalysatoren gemäß Patentanspruch 1, dadurch gekennzeichnet, daß man eine Suspension der Mikroorganismen in einer wässrigen Lösung des Copolymerisats mit dem Alkylendioxydiamin versetzt.

5. Verfahren zur Herstellung von Biokatalysatoren gemäß Patentanspruch 4, dadurch gekennzeichnet, daß es bei einer Temperatur von 0 bis 40° C durchgeführt wird.

6. Verfahren zur Herstellung von Biokatalysatoren gemäß Patentanspruch 4 und 5, dadurch gekennzeichnet, daß das verwendete Copolymerisat hergestellt wird, indem man Acrolein und 1-Vinyl-2-pyrrolidon in wässriger Lösung in Gegenwart eines Initiators bei 10 bis 40° C polymerisiert.

7. Verfahren zur Herstellung von Biokatalysatoren gemäß Patentanspruch 6, dadurch gekennzeichnet, daß man pro Mol 1-Vinyl-2-pyrrolidon 0,5 bis 1,0 Mol Acrolein verwendet.

8. Verfahren zur Herstellung von Biokatalysatoren gemäß Patentanspruch 4 bis 7, dadurch gekennzeichnet, daß das Verhältnis der Oxyaminogruppen des Alkylendioxydiamins und der Aldehydgruppen des Copolymerisats 0,5 bis 1,5 beträgt.

9. Biokatalysatoren gemäß Patentanspruch 1, dadurch gekennzeichnet, daß sie nach dem Verfahren gemäß Patentanspruch 4 bis 8 hergestellt sind.

10. Verwendung von Biokatalysatoren gemäß Patentanspruch 1 bis 3 und 9 zur mikrobiologischen Umwandlung von Steroiden.

11. Verwendung von Biokatalysatoren gemäß Patentanspruch 10, dadurch gekennzeichnet, daß man eine wässrige Suspension des Biokatalysators gemäß Patentanspruch 2 anwendet.

12. Verwendung von Biokatalysatoren gemäß Patentanspruch 11, dadurch gekennzeichnet, daß man der Suspension des Biokatalysators das Steroid in Form einer Lösung oder in mikronisierter Form zusetzt, die Reaktionsmischung bei einer Temperatur von 20 bis 40° C bis zur Umsetzung des Steroids inkubiert und danach den Biokatalysator mittels eines Siebes von der wässrigen Suspension des gebildeten Steroids abtrennt.

**Claims**

1. Biocatalyst containing immobilised microorganisms of the species Arthrobacter simplex, Aspergillus occhraceus, Bacillus sphaericus, Curvularia lunata, Flavobacterium dehydrogenans. Mycobacterium spec. or Saccharomyces uvarum, characterised in that the microorganisms are immobilised in a copolymer of acrolein and 1-vinyl-2-pyrrolidone, which is cross-linked by reaction with an alkyenedioxydiamine of the general formula:
$$H_2NO\text{-}(CH_2)_nONH_2$$
in which n is an integer from 2 to 12.

2. Biocatalyst according to claim 1, characterised in that it contains 10 to 75% by weight microorganisms.

3. Biocatalyst according to claim 1 or 2, characterised in that it has a particle size of 0,05 to 1 mm.

4. Process for the preparation of biocatalysts according to claim 1, characterised in that a suspension of the microorganisms in an aqueous solution of the copolymer is cross-linked with the alkylenedioxydiamine.

5. Process for the preparation of biocatalysts according to claim 4, characterised in that it is carried out at a temperature of 0 to 40°C.

6. Process for the preparation of biocatalysts according to claim 4 or 5, characterised in that the copolymer is prepared by polymerising acrolein and 1-vinyl-2-pyrrolidone in aqueous solution in the presence of an initiator at 10 to 40°C.

7. Process for the preparation of biocatalysts according to claim 6, characterised in that 0.5 to 1.0 mol acrolein per mol 1-vinyl-2-pyrrolidone is used.

8. Process for the preparation of biocatalysts according to any one of claims 4 to 7, characterised in that the proportion of oxyamino groups of the alkylenedioxydiamine to the aldehyde groups of the copolymer is 0.5 to 1.5.

9. Biocatalysts according to claim 1 characterised in that they are prepared by the process according to any one of claims 4 to 8.

10. Use of biocatalysts according to any one of claims 1 to 3 and 9 for the microbiological transformation of steroids.

11. Use of biocatalysts according to claim 10, characterised in an aqueous suspension of the biocatalyst according to claim 2 is used.

12. Use of biocatalysts according to claim 11, characterised in that the suspension of the biocatalyst is added to the steroid in the form of a solution or in micronised form, the reaction mixture is maintained at a temperature of 20 to 40°C until the conversion of the steroid, and the biocatalyst is then separated from the aqueous suspension of the transformed steroid by filtration.

**Revendications**

1. Biocatalyseur contenant des micro-organismes immobilisés de l'espèce Arthrobacter simplex, Aspergillus occhraceus, Bacillus sphaericus, Curvularia lunata, Flavobacterium dehydrogenans, Mycobacterium spec. ou Saccharomyces uvarum, biocatalyseur caractérisé en ce que les microorganismes sont immobilisés dans un copolymère de l'acroléine et de la vinyl-1 pyrrolidone-2 qui a été réticulé par réaction avec une alkylène-dioxy-diamine répondant à la formule générale suivante:
$$H_2NO-(CH_2)_n-ONH_2$$
dans laquelle n représente un nombre de 2 à 12.

2. Biocatalyseur selon la revendication caractérisé en ce qu'il contient de 10 à 75 % en poids de micro-organismes.

3. Biocatalyseur selon l'une des revendications 1 et 2, caractérisé en ce qu'il a une granularité de 0,05 à 1 mm.

4. Procédé de préparation de biocatalyseurs selon la revendication 1, procédé caractérisé en ce qu'on ajoute l'alkylène-dioxy-diamine à une suspension des microorganismes dans une solution aqueuse du copolymère.

5. Procédé de préparation de biocatalyseurs selon la revendication 4, procédé caractérisé en ce qu'on opère à une température de 0 à 40°C.

6. Procédé de préparation de biocatalyseurs selon l'une das revendications 4 et 5, caractérisé en ce qu'on utilise un copolymère qui a été préparé par polymérisation de l'acroléine et de la vinyl-1 pyrrolidone-2 en solution aqueuse, en présence d'un amorceur, à une température de 10 à 40°C.

7. Procédé de préparation de biocatalyseurs selon la revendication 6, caractérisé en ce qu'on utilise de 0,5 à 1,0 mol d'acroléine par mole de vinyl-1 pyrrolidone-2.

8. Procédé de préparation de biocatalyseurs selon l'une des revendications 4 à 7, caractérisé en ce que le rapport des radicaux oxyamino de l'alkylène-dioxy-diamine aux radicaux aldéhydes du copolymère est compris entre 0,5 et 1,5.

9. Biocatalyseurs selon la revendication 1, caractérisé en ce qu'ils ont été préparés par un procédé selon l'une quelconque des revendications 4 à 8.

10. Application de biocatalyseurs selon l'une quelconque des revendications 1, 2, 3 et 9 à la transformation microbiologique de stéroïdes.

11. Application de biocatalyseurs selon la revendication 10, caractérisée en ce qu'on utilise une suspension aqueuse du biocatalyseur selon la revendication 2.

12. Application de biocatalyseurs selon la revendication 11, caractérisée en ce qu'on ajoute le stéroïde sous la forme d'une solution ou à l'état micronisé à la suspension du biocatalyseur, on fait incuber le mélange réactionnel à une température de 20 à 40°C jusqu'à ce que le stéroïde soit transformé, puis on sépare le biocatalyseur, au moyen d'un tamis, de la suspension aqueuse du stéroïde formé.